(19) Europäisches Patentamt / European Patent Office / Office européen des brevets

(11) **EP 2 682 140 A1**

(12) **EUROPEAN PATENT APPLICATION**
published in accordance with Art. 153(4) EPC

(43) Date of publication:
**08.01.2014 Bulletin 2014/02**

(21) Application number: **12755423.6**

(22) Date of filing: **29.02.2012**

(51) Int Cl.:
**A61M 1/36** *(2006.01)*      **A61M 1/34** *(2006.01)*

(86) International application number:
**PCT/JP2012/055043**

(87) International publication number:
**WO 2012/121073 (13.09.2012 Gazette 2012/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.03.2011   JP 2011047556**

(71) Applicants:
- **DIC Corporation**
  **Tokyo 174-8520 (JP)**
- **National University Corporation Hamamatsu University School of Medicine Hamamatsu-shi Shizuoka 431-3192 (JP)**

(72) Inventors:
- **SAKURAI, Naoto**
  **Sakura-shi**
  **Chiba 285-8668 (JP)**
- **IKUSHIMA, Naoya**
  **Sakura-shi**
  **Chiba 285-8668 (JP)**
- **SUZUKI, Tetsuro**
  **Hamamatsu-shi**
  **Shizuoka 431-3192 (JP)**

(74) Representative: **Albrecht, Thomas**
**Kraus & Weisert**
**Patent- und Rechtsanwälte**
**Thomas-Wimmer-Ring 15**
**80539 München (DE)**

(54) **SUGAR-IMMOBILIZED POLYMER SUBSTRATE FOR REMOVING VIRUSES, AND METHOD FOR REMOVING VIRUSES**

(57)     The present invention provides a sugar-immobilized polymer substrate for removing a virus, the polymer substrate allowing efficient removal of a hepatitis virus or the like in a fluid, and a method for removing a virus. In particular, the present invention provides a sugar-immobilized polymer substrate for removing a hepatitis virus or the like, the polymer substrate allowing, in the case of an application to blood of a living body, reduction in the amount of blood taken out of the body, reduction in the removal amount of blood useful components, low invasiveness, and shortening of the operation cycle; and a method for removing a virus. A hollow fiber membrane according to the present invention can be used as a module having a function of effectively removing a virus and a function of not removing useful plasma components.

FIG. 1

EP 2 682 140 A1

## Description

The present invention relates to a sugar-immobilized polymer substrate for removing a virus and a method for removing a virus.

Background Art

Hepatitis C is caused by chronic hepatitis C virus (HCV) infection. In general, the treatment method using medicine for hepatitis C is a combination therapy of pegylated interferon and ribavirin. For patients in which the virus has the genotype 1b and the viral load in the blood is high, the recovery ratio is about 50% and the likelihood of progression to hepatic cirrhosis or liver cancer is high. Accordingly, there has been a demand for the development of a more effective treatment and medicine (Non Patent Literature 1). In general, it is known that a treatment with medicine results in a high recovery ratio in the case of a low viral load in the blood. It has been reported that, when removal of HCV in the blood through a porous filter is combined with a therapy using medicine, the recovery ratio is increased (Non Patent Literature 2). That is, the decrease in the viral load in the body probably resulted in the increase in the recovery ratio.

Patent Literature 1 describes a blood processing apparatus in which a blood inlet, an upstream side blood channel, a plasma separation unit, and a downstream side blood channel are connected in this order; the plasma exit of the plasma separation unit, an upstream side plasma channel, a plasma purifying unit, and a downstream side plasma channel are connected in this order further; and the end of the downstream side plasma channel is connected to a blood-plasma mixing unit provided at an intermediate portion of the downstream side blood channel, wherein at least a blood cell processing unit including a water insoluble carrier for removing a virus and virus-infected cells is provided downstream of the blood-plasma mixing unit of the downstream side blood channel, and the plasma purifying unit is composed of a porous filter membrane having a maximum pore diameter of 20 nm or more and 50 nm or less.

However, this method employing removal with the filter is performed by temporarily achieving separation of blood cells and plasma and then removing a virus from the plasma component; and hence the channel configuration is complicated. Accordingly, there has been a demand for a simpler method of removing a virus from the blood.

A blood-purification adsorbent for hepatitis C virus in which a ligand or the like is immobilized is described in Patent Literature 2. Specifically, a method has been reported in which a peptide that has affinity for immunogloblin or the like and is immobilized on water-insoluble gel is used to efficiently remove immune-complex hepatitis C virus.

On the other hand, it is known that heparin is an effective ligand that binds with HCV (Non Patent Literature 3). Accordingly, by using a substrate in which heparin is immobilized on a polymer support member such as a hollow fiber through which whole blood can be passed without requiring separation of blood cells and plasma, or by using a substrate in which heparin is immobilized in, for example, pores of a blood cell-plasma separation membrane, HCV may be removed more easily. Accordingly, it is expected that, for example, an HCV-removal module that puts a smaller load on patients can be provided.

The substrate on which heparin is immobilized may be in the form of a bead or a porous hollow fiber. Compared with extracorporeal circulation modules filled with particulate heparin-immobilized substrates, internal-circulation or filtration-type extracorporeal circulation modules using porous hollow fibers have fewer portions where the blood stagnates and hence are advantageous in that the configuration is less likely to cause formation of blood clots. In the case of immobilizing heparin on a porous hollow fiber, since the type of surface functional group and the immobilization density vary depending on the material of the substrate, an optimum method needs to be found in accordance with the substrate.

On the other hand, a virus adsorbent having a sugar, specifically, a substrate that adsorbs human immunodeficiency virus (hereafter, referred to as HIV) has been reported. For example, Patent Literature 3 describes an HIV-adsorption polymer substrate having a sugar chain and obtained in the following manner: a polymerizable compound having an ethylenically unsaturated bond and a sugar chain or a polymerizable composition having such a polymerizable compound is brought into contact with a substrate formed of a polymer having methylene groups as the main chain and irradiated with ionizing radiation; or the polymer substrate is irradiated with ionizing radiation and subsequently the polymerizable compound or a polymerizable composition having such a polymerizable compound is brought into contact with the polymer substrate.

Citation List

Patent Literature

PTL 1: Japanese Unexamined Patent Application Publication No. 2005-230165
PTL 2: Japanese Unexamined Patent Application Publication No. 10-323387

PTL 3: Japanese Unexamined Patent Application Publication No. 2010-68910

Non Patent Literature

NPL 1: Viral Hepatitis: Advances in Basic and Clinical Research, Nippon Rinsho, vol. 69, Special Issue vol. 4 (2011)
NPL 2: A. K. Fujiwara et al. Hepatol. Res., 37, 701 (2007) NPL 3: Zahn, J. P. Allain, J. Gen. Virol., 86, 677 (2005)

Summary of Invention

Technical Problem

In view of the related art, an object of the present invention is to provide a substrate or an appliance through which a body fluid such as blood can be passed without causing clogging due to formation of a blood clot or the like, to thereby allow efficient removal of a virus in the body fluid.

Solution to Problem

In order to achieve the object, the inventors of the present invention performed thorough studies on selection of a polymer support member (A) and on how to immobilize, on the polymer support member (A), a sugar that has a function of removing a virus. As a result, the inventors have accomplished the present invention.
Specifically, the present invention relates to a sugar-immobilized polymer substrate for removing a virus, wherein a polymer support member (A) that is surface-treated with a polymer material having a hydroxyl group is bonded to a compound (B) having a group that is capable of reacting with a hydroxyl group to form a covalent bond; and a sugar is immobilized on the compound (B) via a compound (C) having an amino group. The present invention also relates to a polymer substrate for removing a virus, wherein a sugar is immobilized via the compound (B) without using the compound (C).

Advantageous Effects of Invention

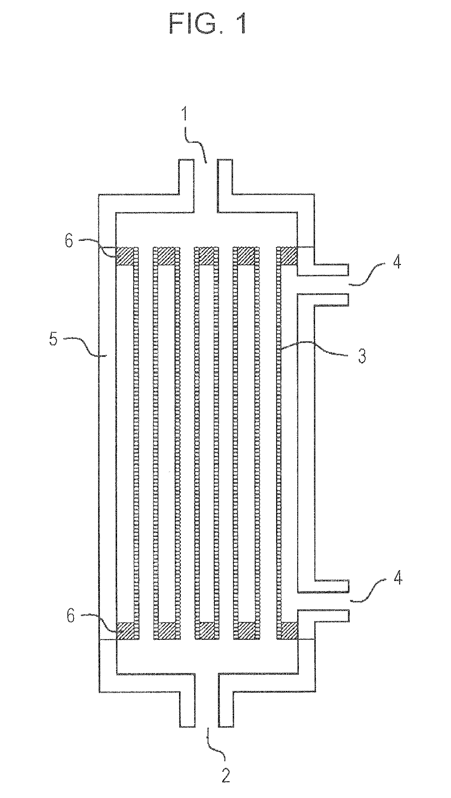

The present invention can provide a polymer substrate that has a function of selectively removing a virus without adsorbing or removing blood components that should not be removed; and a medical appliance using the polymer substrate. Brief Description of Drawing
[Fig. 1] Fig. 1 is a schematic sectional view illustrating a medical appliance including a polymer substrate according to an embodiment of the present invention.

Description of Embodiments

Specifically, the present invention is as follows:

1. A sugar-immobilized polymer substrate for removing a virus, wherein
a polymer support member (A) that is surface-treated with a polymer material having a hydroxyl group is bonded to a compound (B) having a group that is capable of reacting with a hydroxyl group to form a covalent bond; and a sugar is immobilized on the compound (B) via a compound (C) having an amino group,
2. The polymer substrate for removing a virus according to 1., wherein the polymer support member (A) that is surface-treated with a polymer material having a hydroxyl group is a hollow fiber that is surface-treated with an ethylene-vinyl alcohol copolymer, an ethylene-vinyl alcohol-vinyl acetate copolymer, a partially saponified ethylene-vinyl acetate copolymer, a vinyl alcohol-vinyl acetate copolymer, a hydroxymethacrylate copolymer, a partially saponified cellulose acetate, or a glycerin derivative,
3. The polymer substrate for removing a virus according to 2., wherein the hollow fiber is a porous hollow fiber,
4. The polymer substrate for removing a virus according to 2. or 3., wherein the hollow fiber is formed of a material that is polyethylene, polypropylene, or poly-4-methylpentene,
5. The polymer substrate for removing a virus according to 3. or 4., wherein the porous hollow fiber has a mean flow pore size in a range of 50 to 500 nm,
6. The polymer substrate for removing a virus according to any one of 3. to 5., wherein the porous hollow fiber has an inner diameter in a range of 150 to 500 $\mu$m,

7. The polymer substrate for removing a virus according to any one of 3. to 6., wherein the porous hollow fiber has a membrane thickness in a range of 30 to 100 $\mu$m,

8. The polymer substrate for removing a virus according to any one of 3. to 7., wherein an amount of the sugar immobilized on the porous hollow fiber is in a range of 1 to 100 $\mu$g/cm$^2$,

9. The polymer substrate for removing a virus according to any one of 1. to 8., wherein the compound (B) having a group that is capable of reacting with a hydroxyl group to form a covalent bond is epichlorohydrin, a carboxylic anhydride, a dicarboxylic acid compound, a dicarboxylic chloride, a diisocyanate, a diepoxy compound, (meth)acrylic acid, glycidyl (meth)acrylate, or a (meth)acryloyloxyalkyl isocyanate,

10. The polymer substrate for removing a virus according to any one of 1. to 9., wherein the compound (C) having an amino group is polyallylamine, ammonia, 2-aminoethanol, ethylenediamine, butylenediamine, hexamethylenediamine, 1,2-bis(2-aminoethoxy)ethane, 3,3'-diaminodipropylamine, diethylenetriamine, phenylenediamine, or polyethyleneimine,

11. The polymer substrate for removing a virus according to any one of 1. to 10., wherein the sugar is heparin, a heparin derivative in which a primary or secondary hydroxyl group of heparin is subjected to sulfation, a heparin derivative in which an N-acetyl group of heparin is removed and the deacetylated heparin is subjected to N-sulfation, a heparin derivative in which an N-sulfate group of heparin is removed and the desulfated heparin is subjected to N-acetylation, low molecular weight heparin, dextran sulfate, dextran sulfate, fucoidan, chondroitin sulfuric acid A, chondroitin sulfuric acid C, dermatan sulfate, heparinoid, heparan sulfate, rhamnan sulfate, ketaran sulfate, alginic acid, hyaluronic acid, or carboxymethylcellulose,

12. The polymer substrate for removing a virus according to any one of 1. to 11., wherein the virus is hepatitis B virus or hepatitis C virus,

13. A method for removing a virus, using the polymer substrate for removing a virus according to any one of 1. to 12.,

14. The method for removing a virus according to 13., comprising mixing together a fluid having passed through pores of a porous hollow fiber and a fluid not having passed through the pores by passing a fluid containing a virus through the porous hollow fiber, and

15. The method for removing a virus according to 14., wherein the fluid containing a virus is blood containing a virus.

Hereinafter, the present invention will be described in detail.

· Polymer support member (A)

The polymer support member (A) used in the present invention is not particularly limited as long as it is a polymer support member that is surface-treated with a polymer material having a hydroxyl group. The polymer support member (A) may be selected from various polymer support members having high affinity for blood. Examples of the polymer include olefin resins, styrene resins, sulfone resins, acrylic resins, urethane resins, ester resins, ether resins, and cellulose mixed esters. Specific examples include a member that is formed of polyethylene terephthalate, polymethyl methacrylate, polysulfone, polyethersulfone, polyacrylonitrile, polyethylene, polypropylene, poly-4-methylpentene, or the like and that is surface-treated with a polymer material having a hydroxyl group by a publicly known method; and a resin obtained by kneading a resin having a hydroxyl group by a publicly known method in advance.

The form of the polymer support member (A) is not particularly limited and can be selected from various forms such as hollow fibers and beads. In the case of applications to extracorporeal circulation, beads can be used. However, use of beads tends to cause formation of blood clots in stagnation portions. Accordingly, for such applications, hollow fibers are preferably used. The form of hollow fibers can be appropriately selected.

Porous hollow fibers may be produced by a publicly known and commonly used method depending on the usage purpose. In the case of polyolefin hollow fibers, by subjecting spun fibers to an annealing treatment, cold drawing, hot drawing, and heat setting, fibers having various pore sizes and pore-size distributions can be prepared.

When porous hollow fibers are used, a fluid containing a virus is passed through pores of the hollow fibers to thereby efficiently remove the virus. In the case of processing blood during extracorporeal circulation, the whole blood is desirably processed through the pores because of simplicity. However, there are the following problems: the problem of stagnation and the requirement of high biocompatibility because of the direct contact of blood cells with pores.

On the other hand, there is a method in which blood cells and the plasma component are separated from each other and the plasma component alone is passed through the pores to remove a virus from the plasma. In this case, a fluid having passed through the pores of hollow fibers and a fluid not having passed through the pores are generated. Studies on the removal ratio of the virus in the virus-containing fluid have revealed that, regarding the fluid having passed through the pores of hollow fibers, the removal ratio of the virus is high and albumin, which is a useful component in the blood, is not removed. In addition, regarding the fluid not having passed through the pores of hollow fibers, that is, the fluid having come into contact with only the internal surfaces or pores in the region close to the internal surfaces, the removal ratio of the virus is lower than that of the fluid having passed through the pores of hollow fibers; and it has been suggested that removal of the virus mainly occurs when the fluid passes through the pores of porous hollow fibers.

Here, the "fluid passes through the pores" denotes that the fluid passes from the inner surfaces to outer surfaces of hollow fibers or from the outer surfaces to inner surfaces of hollow fibers.

The pore size of such a porous hollow fiber used is not particularly limited as long as it allows efficient removal of the above-described virus. For example, in the case of efficiently removing a virus from plasma in extracorporeal circulation, the design described below is required. In the case of separating blood cells and plasma from each other and removing a virus from the plasma, the function of a plasma separation membrane needs to be provided. Accordingly, in order to prevent entry of blood cell components and blood platelets into the pores, the mean flow pore size is desirably 500 nm or less. Furthermore, because the design needs to be performed such that the permeability of protein components in the plasma is not low, the mean flow pore size is desirably 50 nm or more.

Accordingly, in order to provide the function of a plasma separation membrane, the design needs to be performed to provide a mean flow pore size of 50 to 500 nm. In the present invention, a feature of efficiently removing a virus from plasma having been separated from blood is provided. Thus, in the case of removing a virus, the optimum pore size varies depending of the size of the virus. For example, in the case of hepatitis C virus, the pore size is preferably 80 to 250 nm, more preferably 100 to 180 nm. Alternatively, in the case of a relatively large virus such as human immunodeficiency virus, the pore size is preferably 100 to 250 nm, more preferably 120 to 200 nm.

The inner diameter of a porous hollow fiber used is not particularly limited as long as it allows efficient removal of such a virus. For example, in the case of using a hollow fiber in extracorporeal circulation, the design needs to be performed as described below.

Since the amount of blood taken from the human for circulation is limited, the size of the circulation module or the like cannot be excessively increased. When the inner diameter is large, the number of fibers installed in the module is decreased. Thus, the contact area may be decreased or the linear velocity may become low to cause stagnation of the blood.

On the other hand, when the inner diameter is excessively small, the blood-cell component probably tends to cause clogging. In consideration of the above-described respects, the inner diameter is preferably 150 to 500 $\mu$m, more preferably 160 to 400 $\mu$m, more preferably 170 to 350 $\mu$m.

The membrane thickness of the porous hollow fiber used is not particularly limited as long as it allows efficient removal of the above-described virus. For example, in the case of efficiently removing a virus from plasma in extracorporeal circulation, in consideration of, for example, the plasma separation performance, the contact area, and the mechanical strength of the hollow fiber, the membrane thickness is preferably 30 to 100 $\mu$m, more preferably 35 to 80 $\mu$m, more preferably 40 to 60 $\mu$m.

Furthermore, another substrate having a function of capturing and removing a virus can be attached to an outer portion of the hollow fiber to thereby increase the removal ratio of the virus. Such another substrate is not particularly limited as long as it has a function of capturing and removing a virus. This substrate is, for example, a sugar-chain-immobilized gel or a sugar-chain-immobilized nonwoven fabric.

· Polymer material having hydroxyl group in polymer support member (A)

The polymer material having a hydroxyl group usable in the present invention is, for example, a polymer material containing a vinyl alcohol copolymer such as an ethylene-vinyl alcohol copolymer, an ethylene-vinyl alcohol-vinyl acetate copolymer, a partially saponified ethylene-vinyl acetate copolymer, or a vinyl alcohol-vinyl acetate copolymer; a polymer material containing a hydroxymethacrylate copolymer; a partially saponified cellulose acetate, or a glycerin derivative. Note that, other than the exemplified polymer materials, use of a resin having a hydroxyl group is not particularly limited.

A surface treatment with a polymer material having a hydroxyl group can be performed by a publicly known and commonly used method. For example, a preferred method may be a method of immersing a porous polyolefin in a solution in which a polymer material having a hydroxyl group has been dissolved, withdrawing and then drying the porous polyolefin. In particular, an ethylene-vinyl alcohol copolymer is preferred because a porous polyolefin hollow fiber can be easily made to be hydrophilic as described in, for example, Japanese Unexamined Patent Application Publication No. 61-271003. Alternatively, a technique may be used in which a polyolefin or the like and a polymer material having a hydroxyl group are mixed in advance and the mixture is formed into a porous polyolefin.

· Compound (B) having group that is capable of reacting with hydroxyl group to form covalent bond

A compound (B) having a group that is capable of reacting with a hydroxyl group to form a covalent bond, the compound (B) being usable in the present invention, is not particularly limited as long as the compound (B) has a group that is capable of reacting with a hydroxyl group present in the surface of the surface-treated polymer support member (A), and the compound (B) having been immobilized has, for example, a functional group that easily reacts with an amino group. The compound (B) is, for example, a compound such as epichlorohydrin, a carboxylic anhydride, a dicarboxylic acid, a dicarboxylic chloride, a diisocyanate, or a diepoxy compound; or a compound that can be undergone graft polymerization onto a substrate, such as (meth)acrylic acid, glycidyl (meth)acryl.ate, a (meth)acryloyloxyalkyl isocyanate, or maleic anhydride.

When a compound (B) having a group that is capable of reacting with a hydroxyl group to form a covalent bond is immobilized through a reaction between the compound (B) and a hydroxyl group present in the surface of the polymer support member (A), this immobilization can be performed, for example, under publicly known and commonly used conditions such as conditions for the reaction between a hydroxyl group and an epoxy group; conditions for the reaction between a hydroxyl group and a carboxy group; conditions for the reaction between a hydroxyl group and a carboxylic anhydride; or conditions for the reaction between a hydroxyl group and an isocyanate.

In the case of subjecting a compound having an ethylenically unsaturated group to graft polymerization, a graft polymerization reaction caused by generation of a radical in a polymer material having a hydroxyl group can be used to immobilize (meth)acrylic acid, glycidyl (meth)acrylate, a (meth)acryloyloxyalkyl isocyanate, maleic anhydride, or the like.

A method of generating a radical may be a method of using a reagent such as diammonium cerium nitrate, which is widely used as a radical polymerization initiator for a polymer material having a hydroxyl group, such as cellulose or polyvinyl alcohol.

Note that, in the case where the form of the polymer support member (A) is a hollow fiber and the compound (B) having a group that is capable of reacting with a hydroxyl group to form a covalent bond is immobilized on the hollow fiber, depending on the embodiment, the compound (B) may be immobilized on the inner surface, the outer surface, or both of these surfaces of the fiber. For example, in the case of perfusing blood over the inner surface of a hollow fiber, the compound (B) having a group that is capable of reacting with a hydroxyl group to form a covalent bond can be brought into contact with the inner surface of the hollow fiber to immobilize the compound (B). In contrast, in the case of performing external perfusion, the compound (B) having a group that is capable of reacting with a hydroxyl group to form a covalent bond can be brought into contact with the outer surface of a hollow fiber. In the case where the compound (B) is immobilized in pores and on the inner and outer surfaces of the fiber, for example, a method of making a bundle of fibers and immersing the bundle in a reaction solution, or a method of assembling a module and circulating a reaction solution may be used.

· Compound (C) having amino group

A compound (C) having an amino group used in the present invention is not particularly limited as long as it reacts with the compound (B) having a group that is capable of reacting with a hydroxyl group to form a covalent bond, so that an amino group, which allows easy immobilization of a sugar, is left. Examples of the compound (C) include amines such as polyallylamine, ammonia, 2-aminoethanol, ethylenediamine, butylenediamine, hexamethylenediamine, 1,2-bis(2-aminoethoxy)ethane, 3,3'-diaminodipropylamine, diethylenetriamine, phenylenediamine, and polyethyleneimine. If necessary, an amino group of the compound (C) may have a publicly known and commonly used protecting group that is used for an amino group. The protecting group used is, for example, t-butylcarbamate (Boc group) or benzylcarbamate. Deprotection may also be performed by a publicly known and commonly used method.

The reaction between the compound (C) and the compound (B) having a group that is capable of reacting with a hydroxyl group to form a covalent bond can be appropriately performed under publicly known and commonly used conditions such as conditions for the reaction between an amino group and an epoxy group; conditions for the reaction between an amino group and a carboxy group; conditions for the reaction between an amino group and a carboxylic anhydride; or conditions for the reaction between an amino group and an isocyanate.

· Sugar

A sugar used in the present invention is not particularly limited as long as it can efficiently capture a virus by the action of adsorption or the like to remove the virus from a fluid containing the virus. Such a sugar is, for example, heparin, a heparin derivative in which a primary or secondary hydroxyl group of heparin is subjected to sulfation, a heparin derivative in which an N-acetyl group of heparin is removed and the deacetylated heparin is subjected to N-sulfation, a heparin derivative in which an N-sulfate group of heparin is removed and the desulfated heparin is subjected to N-acetylation, low molecular weight heparin, dextran sulfate (preferably having a sulfur content of 3% to 6%), dextran sulfate (preferably having a sulfur content of 15% to 20%), fucoidan, chondroitin sulfuric acid A, chondroitin sulfuric acid C, dermatan sulfate, heparinoid, heparan sulfate, rhamnan sulfate, ketaran sulfate, alginic acid, hyaluronic acid, or carboxymethylcellulose.

Regarding heparin, in general, publicly known heparin can be used without limitation. Heparin is widely distributed in the body such as the small intestine, the muscle, the lungs, the spleen, and mast cells. Chemically, heparin is a kind of heparan sulfate, which is a glycosaminoglycan. Heparin is a polymer in which $\beta$-D-glucuronic acid or $\alpha$-L-iduronic

acid is polymerized with D-glucosamine through 1,4-bonds. Heparin has a feature of having a very high degree of sulfation, compared with heparan sulfate.

The average molecular weight of heparin is also not particularly limited. However, heparin having a high average molecular weight has low reactivity with the compound (C) and hence the immobilization efficiency of heparin is probably low. Accordingly, the molecular weight of heparin is preferably approximately 500 to 500,000 daltons, more preferably 1,200 to 50,000 daltons, and still more preferably 5,000 to 30,000 daltons.

A heparin derivative used in the present invention is preferably the above-described heparin derivative in which a primary or secondary hydroxyl group of heparin is subjected to sulfation, the above-described heparin derivative in which an N-acetyl group of heparin is removed and the deacetylated heparin is subjected to N-sulfation, or the above-described heparin derivative in which an N-sulfate group of heparin is removed and the desulfated heparin is subjected to N-acetylation.

In the case of synthesizing a heparin derivative in which a primary or secondary hydroxyl group of heparin is subjected to sulfation, for example, an alkali salt of the heparin is passed through an ion-exchange resin (H$^+$) or the like and treated with an amine to thereby prepare heparin amine salt. After that, the heparin amine salt is treated with a sulfating agent to provide the target heparin derivative. The sulfating agent is preferably a publicly known and commonly used sulfating agent such as SO$_3$-pyridine.

In the case of synthesizing a heparin derivative in which an N-acetyl group of heparin is removed and the deacetylated heparin is subjected to N-sulfation, for example, an N-acetyl group of heparin may be deacetylated with hydrazine or the like and then the heparin may be treated with a sulfating agent to provide the target heparin derivative. The sulfating agent is preferably a publicly known and commonly used sulfating agent such as SO$_3$-NMe$_3$.

In the case of synthesizing a heparin derivative in which an N-sulfate group of heparin is removed and the desulfated heparin is subjected to N-acetylation, for example, a pyridinium salt of heparin is prepared, desulfation is then performed in terms of only sulfate groups on nitrogen atoms and N-acetylation is performed by a publicly known and commonly used method.

Regarding low molecular weight heparin, dextran sulfate (having a sulfur content of 3% to 6%), dextran sulfate (having a sulfur content of 15% to 20%), fucoidan, chondroitin sulfuric acid A, chondroitin sulfuric acid C, dermatan sulfate, heparinoid, heparan sulfate, rhamnan sulfate, ketaran sulfate, alginic acid, hyaluronic acid, and carboxymethylcellulose, publicly known and commonly used compounds can be used.

The degree of sulfation of dextran sulfate may be a high degree of sulfation (sulfur content of 15% to 20%) or a low degree of sulfation (sulfur content of 3% to 6%). The degree of sulfation of dextran sulfate is not particularly limited as long as the dextran sulfate can be obtained by a publicly known and commonly used method.

Heparinoid denotes sulfated polysaccharides that are generally described in "The Japanese pharmaceutical codex" and the like. However, the heparinoid is not limited to those described in "The Japanese pharmaceutical codex" as long as it can be obtained by a publicly known and commonly used extraction method or preparation method.

Immobilization of a sugar with the compound (C) having an amino group requires bonding of the compound (C) and the sugar through a covalent bond. Such a bond can be formed by appropriately performing a publicly known and commonly used reaction.

A preferred reaction for immobilization of a sugar may be an amidation reaction or a reduction amination reaction. Regarding an amidation method, a publicly known and commonly used amidation reaction used for peptide synthesis or the like may be appropriately performed: for example, amidation with an active ester, amidation with a condensing agent, the combination of the foregoing, a mixed anhydride method, an azide method, an oxidation-reduction method, a DPPA method, or a Woodward method. The reduction amination reaction can be performed by a publicly known and commonly used method in which the reaction between an amino group of the compound (C) and the reducing terminal of a sugar is caused.

Amidation with an active ester may be performed by the following method: an active ester in which a highly cleavable group is temporarily condensed with a carboxy group is formed with, for example, NHS (N-hydroxysuccinimide), nitrophenol, pentafluorophenol, DMAP (4-dimethylaminopyridine), HOBT (1-hydroxybenzotriazole), HOAT (hydroxyazabenzotriazole), or the like; and the active ester is caused to react with an amino group. Although amidation with a condensing agent may be performed alone, this amidation may be performed in combination with the active ester. Examples of the condensing agent include EDC (1-(3-dimethylaminopropyl-3-ethyl-carbodiimidehydrochloride), HONB (endo-N-hydroxy-5-norbornene-2,3-dicarboxamide), DCC (dicyclohexylcarbodiimide), BOP (benzotriazole-1-yloxytris (dimethylamino)phosphonium hexafluorophosphate), HBTU (O-benzotriazole-1-yl-N,N,N',N'-tetramethyluronium hexafluorophosphate), TBTU (O-benzotriazole-1-yl-N,N,N',N'-tetramethyluronium tetrafluoroborate), HOBt (1-hydroxybenzotriazole), HOOBt (3,4-dihydro-3-hydroxy-4-oxo-1,2,3-benzotriazine), di-p-trioylcarbodiimide, DIC (diisopropylcarbodiimide), BDP (1-benzotriazolediethylphosphate-1-cyclohexyl-3-(2-morpholinylethyl)carbodiimide), cyanuric fluoride, cyanuric chloride, TFFH (tetramethylfluoroformamidinium hexafluorophosphosphate), DPPA (diphenylphosphorazidate), TSTU (O-(N-succinimidyl)-N,N,N',N'-tetramethyluronium tetrafluoroborate), HATU (N-[(dimethylamino)-1-H-1,2,3-triazolo[4,5,6]-pyridin-1-ylmethylene]-N-methylmethaneaminium hexafluorophosphate N-oxide), BOP-Cl (bis(2-oxo-3-oxazolidinyl)phosphine chloride), PyBOP ((1-H-1,2,3-benzotriazole-1-yloxy)-tris(pyrrolidino)phosphonium tetrafluorophosphate), BrOP (bromotris(dimethylamino)phosphonium hexafluorophosphate), DEPBT (3-(diethoxyphosphoryloxy)-1,2,3-benzotriazin-4(3H)-one), and PyBrOP (bromotris(pyrrolidino)phosphonium hexafluorophosphate).

Solvents that can be used in such an amidation method may be water and organic solvents that can be used for peptide synthesis. Examples of the solvents include dimethylformamide (DMF), dimethyl sulfoxide (DMSO), hexaphosphoroamide, dioxane, tetrahydrofuran (THF), ethyl acetate, solvent mixtures of the foregoing, and aqueous solutions containing the foregoing.

In addition to immobilization with the compound (C) having an amino group, a sugar can also be immobilized with the compound (B) having a group that is capable of reacting with a hydroxyl group to form a covalent bond. Selection may be performed in accordance with the usage purpose, reaction conditions, equipment, or the like.

The amount of a sugar immobilized is not particularly limited as long as a virus can be efficiently removed. Note that, in the case of extracorporeal circulation, biocompatibility is important. Accordingly, the amount needs to be adjusted such that, for example, adsorption of plasma protein and activation of the complement do not occur. In this case, the immobilization amount can be adjusted by, for example, a method of adjusting the introduction amount of the compound (C) having an amino group or a method of changing conditions for the sugar-immobilization reaction. Studies have revealed that the amount may be 1 to 100 $\mu g/cm^2$, preferably 2 to 80 $\mu g/cm^2$, more preferably 3 to 70 $\mu g/cm^2$.

· Fluid containing virus

A target fluid containing a virus in the present invention is not particularly limited as long as it is a fluid containing a virus. Specifically, this fluid may be, for example, a body fluid, which is a liquid component in the human body, or a culture fluid containing a virus. Specific examples of the body fluid include blood, saliva, perspiration, urine, snivel, semen, plasma, lymph, and tissue fluid.

The form of a medical appliance including a polymer substrate according to the present invention is not particularly limited as long as the form is usable in the above-described applications. Examples of the form include a hollow fiber module, a filtration column, and a filter. In such a hollow fiber module or a filtration column, the form and material of the container are not particularly limited. In the case of application to extracorporeal circulation for a body fluid (blood), preferred is a cylindrical container having an internal volume of 10 to 400 mL and an outer diameter of about 2 to about 10 cm, and more preferred is a cylindrical container having an internal volume of 20 to 300 mL and an outer diameter of about 2.5 to about 7 cm. An example of the appliance is illustrated in Fig. 1.

A method of using a medical appliance according to the present invention may be any method as long as it allows contact of the appliance with the fluid containing a virus to remove and separate the virus in the fluid.

EXAMPLES

The present invention will be described further in detail with reference to Examples described below.

<Pore size of porous polymer substrate>

The mean flow pore size (the mean pore size of recessed portions of pores extending from one side to the other side of a membrane) was measured in compliance with ASTM F316-86 and ASTM E1294-89 with a "Perm-Porometer CFP-200AEX" manufactured by Porous Materials, Inc. by a half-dry method. The test solution used was perfluoropolyester (tradename "Galwick").

It is not necessary for such a pore to extend through the membrane as a straight tube. The pore may bend within the membrane. Some pores may be integrated within the membrane. Conversely, a single pore may be branched. Such structures may be simultaneously present.

<Amount of sugar immobilized>

The amount of a sugar immobilized on a hollow fiber was calculated from the dye adsorption amount of 1,9-dimethylmethylene blue. Formation of calibration curve: a dye aqueous solution was prepared and mixed with a predetermined amount of the sugar to form a sugar-dye complex; this solution is mixed with hexane to separate the sugar-dye complex from the aqueous phase; the amount of the dye remaining in the aqueous solution was measured in terms of absorbance (650 nm); and the amount of the sugar added and the absorbance were used to form a calibration curve. Measurement of sample: in a dye solution, a hollow fiber was immersed for a predetermined period; and the dye adsorption amount was used to calculate the amount of the sugar immobilized.

<HCV removal test>

A hollow fiber module having a membrane area of 1.8 cm$^2$ was prepared. The plasma (untreated fluid, 0.6 mL) of an HCV patient was passed through the module to provide a fluid (filtrate, 0.3 mL) having passed through the pores and a fluid (internal solution, 0.3 mL) not having passed through the pores. The sample was measured with an Ortho HCV antigen ELISA test. The HCV removal ratio (%) was calculated with the following formula:

$$\text{HCV removal ratio (\%)} = (1 - \text{HCV load in filtrate/HCV load in untreated fluid}) \times 100.$$

<ELISA>

The sample is pretreated with a pretreatment solution (SDS) so that the HCV core antigen is released and the HCV antibody present therewith is simultaneously deactivated. Thus, a measurement sample is prepared. The measurement sample is placed on an HCV core antigen-antibody immobilization plate and incubated. After the reaction proceeds for a predetermined time, the sample is rinsed, mixed with HCV core antigen-antibody labeled with horeradish peroxidase, and incubated. After the reaction proceeds for a predetermined time, the sample is rinsed, mixed with an o-phenylenediamine reagent, and incubated. After the reaction proceeds for a predetermined time, a quenching solution is added. The color development is measured by photometry at a wavelength of 492 nm. From the absorbance of the sample, the concentration is calculated.

<Permeation amount of plasma albumin>

The sample is mixed with a bromocresol green reagent. The color development is measured by photometry at a wavelength of 630 nm. From the absorbance of the sample, the concentration was calculated.

$$\text{Permeation ratio of albumin (\%)} = (\text{amount of albumin in filtrate/amount of albumin in untreated fluid}) \times 100$$

Preparation example of polymer support member (A)

A high-density polyethylene having a density of 0.968 g/cm$^3$ and a melt index of 5.5 (HIZEX 2200J, manufactured by Mitsui Petrochemicals Industries, Ltd.) was spun with a hollow-fiber-forming spinneret having an extrusion orifice diameter of 16 mm, an annular slit width of 2.5 mm, and an extrusion cross section of 1.06 cm$^2$ at a spinning temperature of 160°C, and was wound up at a spinning draft of 1427. The dimensions of the resultant undrawn hollow fiber were an inner diameter of 308 $\mu$m and a membrane thickness of 64 $\mu$m.
The undrawn hollow fiber was heat-treated at 115°C for 24 hours while being kept at a constant length. Subsequently, the fiber was subjected to drawing with a draw ratio of 1.8 at room temperature at a deformation rate of 21400%/min, then to hot drawing in a heating furnace at 100°C at a deformation rate of 330%/min until the total draw ratio reached 4.8, and further continuously to heat shrinkage in a heating furnace at 125°C until the total draw ratio reached 2.8. Thus, a drawn fiber was obtained. Subsequently, an ethylene-vinyl alcohol copolymer (EVOH) having an ethylene content of 44% was heated and dissolved in 75% ethanol aqueous solution to provide a solution having a concentration of 2.5% by weight. The drawn fiber was immersed for 100 seconds in this solution kept at a temperature of 50°C, kept in a 50°C ethanol saturated vapor at the temperature for 80 seconds, and further dried to remove the solvent for 80 seconds. The resultant EVOH-hydrophilized porous hollow fiber membrane had an inner diameter of 287 $\mu$m and a membrane thickness of 52 $\mu$m.

(Example 1)

On the basis of Japanese Unexamined Patent Application Publication No. 2-029260, a test tube was charged with 26 mL of acetone, 21 mL of epichlorohydrin, and 5 mL of 40% NaOH aqueous solution; and, into this mixture, the EVOH-hydrophilized porous hollow fiber membrane having an inner diameter of 287 $\mu$m, a membrane thickness of 52

**EP 2 682 140 A1**

$\mu$m, and a mean pore size of 102 nm was immersed. Ultrasonic waves were applied to cause a reaction at 30°C to 40°C for 5 hours. After the reaction was completed, washing with acetone and water was performed to provide a hollow fiber into which an epoxy group had been introduced. Regarding the amount of an epoxy group introduced, a $Na_2S_2O_3$ solution was used and titration with NaOH was performed. As a result, it was found that an epoxy group was introduced in about 0.01 $\mu$mol/cm$^2$.

Subsequently, the epoxy-group-introduced hollow fiber was immersed in 28% aqueous ammonia and was caused to react at room temperature overnight. After the reaction was completed, washing with water was performed to provide a hollow fiber into which a primary amino group had been introduced. The amount of an amino group introduced was determined by titration with HCl and was found to be about 0.03 $\mu$mol/cm$^2$.

Subsequently, to a test tube, 400 mg of heparin and 50 mg of sodium cyanoborohydride were placed and dissolved in 50 mL of PBS; into this solution, the amino-group-introduced hollow fiber was immersed and was caused to react at 40°C for 3 days. After the reaction was completed, washing with a saturated saline solution and water was performed. In order to acetylate the remaining amino group, the hollow fiber was placed in 36 mL of AcONa aqueous solution (0.2 mol/L) having a methanol content of 50% and cooled with ice. While the cooling with ice was performed, 18 mL of acetic anhydride was dropped and, in this state, ultrasonic waves were used to cause a reaction for 30 minutes.

Furthermore, the reaction was caused to proceed in a water bath (10°C to 25°C) for 30 minutes. After the reaction was completed, washing with water was performed and the same acetylation treatment was performed again. After the reaction was completed, washing with a saturated saline solution, water, and PBS was performed and a heparin-immobilized hollow fiber was obtained. The immobilization amount was measured on the basis of the adsorption amount of methylene blue. As a result, the immobilization amount was 17 $\mu$g/cm$^2$ (in terms of inner surface area).

A module obtained by using the above-described heparin-immobilized hollow fiber membrane was used to filtrate the plasma of an HCV patient. As a result, 76% of the HCV was removed. At this time, the permeation ratio of albumin was 99% or more.

(Comparative example 1)

The same evaluation was performed with the EVOH-hydrophilized hollow fiber membrane used in Example 1 and not including the immobilized sugar. As a result, the removal ratio of HCV was 64%. Accordingly, it has been demonstrated that the heparin-immobilized hollow fiber can be made to have a high removal ratio of HCV without causing removal of living body components such as albumin.

(Example 2)

Heparin was immobilized by the same procedures as in Example 1 except that the EVOH-hydrophilized membrane in Example 1 was changed so as to have an inner diameter of 267 $\mu$m, a membrane thickness of 54 $\mu$m, and a mean pore size of 246 nm. The immobilization amount was measured. As a result, the immobilization amount was 17 $\mu$g/cm$^2$ (in terms of inner surface area).

A module obtained by using the above-described heparin-immobilized hollow fiber membrane was used to filtrate the plasma of an HCV patient. As a result, 48% of the HCV was removed. At this time, the permeation ratio of albumin was 98%.

(Comparative example 2)

The same evaluation was performed with the EVOH-hydrophilized hollow fiber membrane used in Example 2 and not including the immobilized sugar. As a result, the removal ratio of HCV was 33%.

(Example 3)

Heparin was immobilized by the same procedures as in Example 1 except that the EVOH-hydrophilized membrane in Example 1 was changed so as to have an inner diameter of 300 $\mu$m, a membrane thickness of 40 $\mu$m, and a mean pore size of 226 nm. The immobilization amount was measured. As a result, the immobilization amount was 15 $\mu$g/cm$^2$ (in terms of inner surface area).

A module obtained by using the above-described heparin-immobilized hollow fiber membrane was used to filtrate the plasma of an HCV patient. As a result, 59% of the HCV was removed. At this time, the permeation ratio of albumin was 99% or more.

(Comparative example 3)

The same evaluation was performed with the EVOH-hydrophilized hollow fiber membrane used in Example 3 and not including the immobilized sugar. As a result, the  removal ratio of HCV was 37%.

(Example 4)

Heparin was immobilized by the same procedures as in Example 1 except that the EVOH-hydrophilized membrane in Example 1 was changed so as to have an inner diameter of 257 $\mu$m, a membrane thickness of 54 $\mu$m, and a mean pore size of 183 nm. The immobilization amount was measured. As a result, the immobilization amount was 18 $\mu$g/cm$^2$ (in terms of inner surface area).
A module obtained by using the above-described heparin-immobilized hollow fiber membrane was used to filtrate the plasma of an HCV patient. As a result, 69% of the HCV was removed. At this time, the permeation ratio of albumin was 99% or more.

(Comparative example 4)

The same evaluation was performed with the EVOH-hydrophilized hollow fiber membrane used in Example 4 and not including the immobilized sugar. As a result, the removal ratio of HCV was 35%.

(Example 5)

Heparin was immobilized by the same procedures as in Example 1 except that the EVOH-hydrophilized membrane in Example 1 was changed so as to have an inner diameter of 176$\mu$ m, a membrane thickness of 40 $\mu$m, and a mean pore size of 153 nm. The immobilization amount was measured. As a result, the immobilization amount was 16 $\mu$g/cm$^2$ (in terms of inner surface area). A module obtained by using the above-described heparin-immobilized hollow fiber membrane was used to filtrate the plasma of an HCV patient. As a result, 59% of the HCV was removed. At this time, the permeation ratio of albumin was 99% or more.

(Comparative example 5)

The same evaluation was performed with the EVOH-hydrophilized hollow fiber membrane used in Example 5 and not including the immobilized sugar. As a result, the removal ratio of HCV was 31%.

(Example 6)

Heparin was immobilized by the same procedures as in Example 1 except that the EVOH-hydrophilized membrane in Example 1 was changed so as to have an inner diameter of 168 $\mu$m, a membrane thickness of 63 $\mu$m, and a mean pore size of 118 nm. The immobilization amount was measured. As a result, the immobilization amount was 17 $\mu$g/cm$^2$ (in terms of inner surface area).
A module obtained by using the above-described heparin-immobilized hollow fiber membrane was used to filtrate the plasma of an HCV patient. As a result, 67% of the HCV was  removed. At this time, the permeation ratio of albumin was 96%.

(Comparative example 6)

The same evaluation was performed with the EVOH-hydrophilized hollow fiber membrane used in Example 6 and not including the immobilized sugar. As a result, the removal ratio of HCV was 45%.

(Example 7)

Heparin was immobilized by the same procedures as in Example 1 except that the EVOH-hydrophilized membrane in Example 1 was changed so as to have an inner diameter of 170 $\mu$m, a membrane thickness of 54 $\mu$m, and a mean pore size of 117 nm. The immobilization amount was measured. As a result, the immobilization amount was 20 $\mu$g/cm$^2$ (in terms of inner surface area).
A module obtained by using the above-described heparin-immobilized hollow fiber membrane was used to filtrate the plasma of an HCV patient. As a result, 67% of the HCV was removed. At this time, the permeation ratio of albumin was 97%.

(Comparative example 7)

The same evaluation was performed with the EVOH-hydrophilized hollow fiber membrane used in Example 7 and not including the immobilized sugar. As a result, the  removal ratio of HCV was 55%.

(Example 8)

Low molecular weight heparin was immobilized by the same procedures as in Example 4 except that the heparin in Example 4 was changed to low molecular weight heparin. The immobilization amount was measured. As a result, the immobilization amount was 21 $\mu$g/cm$^2$ (in terms of inner surface area).
A module obtained by using the above-described low-molecular-weight-heparin-immobilized hollow fiber membrane was used to filtrate the plasma of an HCV patient. As a result, 51% of the HCV was removed. At this time, the permeation ratio of albumin was 99% or more.

(Comparative example 8)

The same evaluation was performed with the EVOH-hydrophilized hollow fiber membrane used in Example 8 and not including the immobilized sugar. As a result, the removal ratio of HCV was 35%. Accordingly, it has been demonstrated that the low-molecular-weight-heparin-immobilized hollow fiber can be made to have a high removal ratio of HCV without causing removal of living body components such as albumin.

(Example 9)

A dextran sulfate was immobilized by the same procedures as in Example 4 except that the heparin in Example 4 was changed to a dextran sulfate having a molecular weight of 5000 (a degree of sulfation of 15% to 20%). The immobilization amount was measured. As a result, the immobilization amount was 8 $\mu$g/cm$^2$ (in terms of inner surface area).
A module obtained by using the above-described dextran-sulfate-immobilized hollow fiber membrane was used to filtrate the plasma of an HCV patient. As a result, 49% of the HCV was removed. At this time, the permeation ratio of albumin was 99% or more.

(Comparative example 9)

The same evaluation was performed with the EVOH-hydrophilized hollow fiber membrane used in Example 9 and not including the immobilized sugar. As a result, the removal ratio of HCV was 35%. Accordingly, it has been demonstrated that the hollow fiber on which a dextran sulfate having a molecular weight of 5000 (a degree of sulfation of 15% to 20%) is immobilized can be made to have a high removal ratio of HCV without causing removal of living body components such as albumin.

(Example 10)

A dextran sulfate was immobilized by the same  procedures as in Example 4 except that the heparin in Example 4 was changed to a dextran sulfate having a molecular weight of 20000 (a degree of sulfation of 15% to 20%). The immobilization amount was measured. As a result, the immobilization amount was 10 $\mu$g/cm$^2$ (in terms of inner surface area).
A module obtained by using the above-described dextran-sulfate-immobilized hollow fiber membrane was used to filtrate the plasma of an HCV patient. As a result, 49% of the HCV was removed. At this time, the permeation ratio of albumin was 99% or more.

(Comparative example 10)

The same evaluation was performed with the EVOH-hydrophilized hollow fiber membrane used in Example 10 and not including the immobilized sugar. As a result, the removal ratio of HCV was 35%. Accordingly, it has been demonstrated that the hollow fiber on which a dextran sulfate having a molecular weight of 20000 (a degree of sulfation of 15% to 20%) is immobilized can be made to have a high removal ratio of HCV without causing removal of living body components such as albumin.

(Example 11)

Heparin was immobilized by the same procedures as in Example 2 except that the 28% aqueous ammonia in Example 2 was changed to 5% polyallylamine aqueous solution. The immobilization amount was measured. As a result, the immobilization amount was 43 $\mu$g/cm$^2$ (in terms of inner surface area).
A module obtained by using the above-described heparin-immobilized hollow fiber membrane was used to filtrate the plasma of an HCV patient. As a result, 59% of the HCV was removed. At this time, the permeation ratio of albumin was 99%.

(Comparative example 11)

The same evaluation was performed with the EVOH-hydrophilized hollow fiber membrane used in Example 11 and not including the immobilized sugar. As a result, the removal ratio of HCV was 33%.

(Example 12)

Heparin was immobilized by the same procedures as in Example 11 except that the EVOH-hydrophilized membrane in Example 11 was changed so as to have an inner diameter of 257 $\mu$m, a membrane thickness of 54 $\mu$m, and a mean pore size of 183 nm. The immobilization amount was measured. As a result, the immobilization amount was 46 $\mu$g/cm$^2$ (in terms of inner surface area).
A module obtained by using the above-described heparin-immobilized hollow fiber membrane was used to filtrate the plasma of an HCV patient. As a result, 59% of the HCV was removed. At this time, the permeation ratio of albumin was 98%.

(Comparative example 12)

The same evaluation was performed with the EVOH-hydrophilized hollow fiber membrane used in Example 12 and not including the immobilized sugar. As a result, the removal ratio of HCV was 35%.

(Example 13)

Low molecular weight heparin was immobilized by the same procedures as in Example 12 except that the heparin in Example 12 was changed to low molecular weight heparin. The immobilization amount was measured. As a result, the immobilization amount was 70 $\mu$g/cm$^2$ (in terms of inner surface area).
A module obtained by using the above-described low-molecular-weight-heparin-immobilized hollow fiber membrane was used to filtrate the plasma of an HCV patient. As a result, 59% of the HCV was removed. At this time, the permeation ratio of albumin was 99% or more.

(Example 14)

A dextran sulfate was immobilized by the same procedures as in Example 12 except that the heparin in Example 12 was changed to a dextran sulfate having a molecular weight of 5000 (a sulfur content of 15% to 20%). The immobilization amount was measured. As a result, the immobilization amount was 30 $\mu$g/cm$^2$ (in terms of inner surface area).
A module obtained by using the above-described dextran-sulfate-immobilized hollow fiber membrane was used to filtrate the plasma of an HCV patient. As a result, 58% of the HCV was removed. At this time, the permeation ratio of albumin was 99% or more.

(Example 15)

A dextran sulfate was immobilized by the same procedures as in Example 12 except that the heparin in Example 12 was changed to a dextran sulfate having a molecular weight of 20000 (a sulfur content of 15% to 20%). The immobilization amount was measured. As a result, the immobilization amount was 34 $\mu$g/cm$^2$ (in terms of inner surface area).
A module obtained by using the above-described dextran-sulfate-immobilized hollow fiber membrane was used to filtrate the plasma of an HCV patient. As a result, 67% of the HCV was removed. At this time, the permeation ratio of albumin was 99%.

(Example 16)

To a test tube, 3.5 g of succinic anhydride and 350 uL of triethylamine were placed and dissolved in 30 mL of acetone. Into this mixture, the EVOH-hydrophilized hollow fiber membrane used in Example 4 was immersed and was caused to react at room temperature for 24 hours. After the reaction was completed, washing with acetone and water was performed. Vacuum drying was performed to provide a hollow fiber into which a carboxyl group had been introduced.
To a test tube, 19 mg of EDC (0.1 mmol) and 12 mg of NHS (0.1 mmol) were placed and dissolved in 30 mL of PBS; into this solution, the carboxyl-group-introduced hollow fiber was immersed and was caused to react for 5 minutes. Into this solution, 1 mL of 20% polyallylamine aqueous solution was dropped and a reaction was caused to proceed at room temperature for 4 hours. After the reaction was completed, washing with water was performed to provide a hollow fiber into which a primary amino group had been introduced. After that, as in Example 1, heparin was immobilized and the acetylation treatment of an amino group was performed to provide a heparin-immobilized hollow fiber. The immobilization amount was measured. As a result, the immobilization amount was 66 $\mu$g/cm$^2$ (in terms of inner surface area).
A module obtained by using the above-described heparin-immobilized hollow fiber membrane was used to filtrate the plasma of an HCV patient. As a result, 64% of the HCV was removed. At this time, the permeation ratio of albumin was 98%.

(Example 17)

Low molecular weight heparin was immobilized by the same procedures as in Example 16 except that the heparin in Example 16 was changed to low molecular weight heparin. The immobilization amount was measured. As a result, the immobilization amount was 68 $\mu$g/cm$^2$ (in terms of inner surface area).
A module obtained by using the above-described heparin-immobilized hollow fiber membrane was used to filtrate the plasma of an HCV patient. As a result, 53% of the HCV was removed. At this time, the permeation ratio of albumin was 99%.

(Example 18)

Heparin was immobilized by the same procedures as in Example 4 except that the reaction of immobilizing heparin on an amino group performed by the reduction amination was performed by an active-ester-formation condensation reaction with EDC. The immobilization amount was measured. As a result, the immobilization amount was 30 $\mu$g/cm$^2$ (in terms of inner surface area).
A module obtained by using the above-described heparin-immobilized hollow fiber membrane was used to filtrate the plasma of an HCV patient. As a result, 56% of the HCV was removed. At this time, the permeation ratio of albumin was 94%.

(Example 19)

After an epoxy group was introduced into the EVOH-hydrophilized hollow fiber membrane used in Example 4 by the same procedures as in Example 1, the membrane was immersed in 10% heparin aqueous solution and caused to react at 40°C. The immobilization amount was measured. As a result, the immobilization amount was 11 $\mu$g/cm$^2$ (in terms of inner surface area).
A module obtained by using the above-described heparin-immobilized hollow fiber membrane was used to filtrate the plasma of an HCV patient. As a result, 41% of the HCV was removed. At this time, the permeation ratio of albumin was 99%.

(Example 20)

A dextran sulfate having a low degree of sulfation was immobilized by the same procedures as in Example 4 except that the heparin in Example 4 was changed to a dextran sulfate (having a sulfur content of 3% to 6%). The immobilization amount was measured. As a result, the immobilization amount was 41 $\mu$g/cm$^2$ (in terms of inner surface area).
A module obtained by using the above-described dextran sulfate having a low degree of sulfation was used to filtrate the plasma of an HCV patient. As a result, 40% of the HCV was removed. At this time, the permeation ratio of albumin was 99% or more.

(Comparative example 13)

The same evaluation was performed with the EVOH-hydrophilized hollow fiber membrane used in Example 20 and not including the immobilized sugar. As a result, the removal ratio of HCV was 30%. Accordingly, it has been demonstrated that the hollow fiber on which a dextran sulfate having a low degree of sulfation is immobilized can be made to have a high removal ratio of HCV without causing removal of living body components such as albumin.

(Example 21)

Heparinoid was immobilized by the same procedures as in Example 4 except that the heparin in Example 4 was changed to heparinoid. The immobilization amount was measured. As a result, the immobilization amount was 18 $\mu$g/cm$^2$ (in terms of inner surface area).
A module obtained by using the above-described heparinoid-immobilized hollow fiber membrane was used to filtrate the plasma of an HCV patient. As a result, 62% of the HCV was removed. At this time, the permeation ratio of albumin was 99% or more.

(Comparative example 14)

The same evaluation was performed with the EVOH-hydrophilized hollow fiber membrane used in Example 21 and not including the immobilized sugar. As a result, the removal ratio of HCV was 30%. Accordingly, it has been demonstrated that the heparinoid-immobilized hollow fiber can be made to have a high removal ratio of HCV without causing removal of living body components such as albumin.

(Example 22) Blood cell-plasma separation test

Needles for extracting plasma (15-gauge dual-threaded plastic needles, manufactured by Musashi Engineering, Inc.) were attached to side surface portions of both ends of a housing pipe for a hollow fiber module (having an inside diameter of 3 mm, an outside diameter of 5 mm, and a full length of 225 mm). In this pipe, 37 porous hollow fiber membranes prepared in Example 1 were inserted. Both end portions of the membranes were fixed on the housing pipe with a 5-minute-type epoxy resin adhesive (Bond Quick Set, manufactured by Konishi Co., Ltd.). In addition, blood inflow and outflow connectors (Luer fitting, manufactured by ISIS Co., Ltd.) were attached to both end portions. Thus, a hollow fiber module having an effective length of 15 cm and an effective filtration area of 50 cm$^2$ was obtained. Blood was collected from a healthy volunteer and mixed with an anticoagulant solution (CPD solution, citrate-phosphate-dextrose solution) to provide an anticoagulant blood. This blood was mixed with the plasma of an HCV patient to provide a blood for evaluation of the capability of removing the virus. The HCV-containing blood (16 mL) was placed in a reservoir and heated to 37°C. The blood was passed through the hollow fiber module at a blood flow rate of 0.33 mL/min and plasma separation was performed at a plasma filtration flow rate of 0.1 mL/min. After 30 minutes elapsed, the viral load of the separated plasma was measured and 80% of the virus was found to be removed. At this time, the permeation ratio of albumin was 99% or more and the permeation ratio of LDL was 89%. Thus, it was demonstrated that the virus can be efficiently removed without removing useful plasma components. During the test, an increase in TMP or the like due to clogging or noticeable hemolysis was not observed. This has revealed the following: by using a ligand-immobilized hollow fiber membrane module, while plasma separation is easily and continuously performed, the virus can be simultaneously removed.

Industrial Applicability

A polymer substrate according to the present invention can be applied to a virus removal appliance and this appliance can be used for removing a virus. Reference Signs List
1:   virus fluid inflow port
2:   outflow port for fluid not having passed through pores
3:   hollow fiber membrane
4:   outflow port for virus fluid having passed through pores
5:   container
6:   partition

**Claims**

1.  A sugar-immobilized polymer substrate for removing a virus, wherein a polymer support member (A) that is surface-treated with a polymer material having a hydroxyl group is bonded to a compound (B) having a group that is capable of reacting with a hydroxyl group to form a covalent bond; and a sugar is immobilized on the compound (B) via a compound (C) having an amino group.

2.  The polymer substrate for removing a virus according to Claim 1, wherein the polymer support member (A) that is surface-treated with a polymer material having a hydroxyl group is a hollow fiber that is surface-treated with an ethylene-vinyl alcohol copolymer, an ethylene-vinyl alcohol-vinyl acetate copolymer, a partially saponified ethylene-vinyl acetate copolymer, a vinyl alcohol-vinyl acetate copolymer, a hydroxymethacrylate copolymer, a partially saponified cellulose acetate, or a glycerin derivative.

3.  The polymer substrate for removing a virus according to Claim 2, wherein the hollow fiber is a porous hollow fiber.

4.  The polymer substrate for removing a virus according to Claim 3, wherein the hollow fiber is formed of a material that is polyethylene, polypropylene, or poly-4-methylpentene.

5.  The polymer substrate for removing a virus according to Claim 3 or 4, wherein the porous hollow fiber has a mean flow pore size in a range of 50 to 500 nm.

6.  The polymer substrate for removing a virus according to any one of Claims 3 to 5, wherein the porous hollow fiber has an inner diameter in a range of 150 to 500 $\mu$m.

7.  The polymer substrate for removing a virus according to any one of Claims 3 to 6, wherein the porous hollow fiber has a membrane thickness in a range of 30 to 100 $\mu$m.

8.  The polymer substrate for removing a virus according to any one of Claims 3 to 7, wherein an amount of the sugar immobilized on the porous hollow fiber is in a range of 1 to 100 $\mu$g/cm$^2$.

9.  The polymer substrate for removing a virus according to any one of Claims 1 to 8, wherein the compound (B) having a group that is capable of reacting with a hydroxyl group to form a covalent bond is epichlorohydrin, a carboxylic anhydride, a dicarboxylic acid compound, a dicarboxylic chloride, a diisocyanate, a diepoxy compound, (meth) acrylic acid, glycidyl (meth)acrylate, or a (meth)acryloyloxyalkyl isocyanate.

10. The polymer substrate for removing a virus according to any one of Claims 1 to 9, wherein the compound (C) having an amino group is polyallylamine, ammonia, 2-aminoethanol, ethylenediamine, butylenediamine, hexamethylenediamine, 1,2-bis(2-aminoethoxy)ethane, 3,3'-diaminodipropylamine, diethylenetriamine, phenylenediamine, or polyethyleneimine.

11. The polymer substrate for removing a virus according to any one of Claims 1 to 10, wherein the sugar is heparin, a heparin derivative in which a primary or secondary hydroxyl group of heparin is subjected to sulfation, a heparin derivative in which an N-acetyl group of heparin is removed and the deacetylated heparin is subjected to N-sulfation, a heparin derivative in which an N-sulfate group of heparin is removed and the desulfated heparin is subjected to N-acetylation, low molecular weight heparin, dextran sulfate, dextran sulfate, fucoidan, chondroitin sulfuric acid A, chondroitin sulfuric acid C, dermatan sulfate, heparinoid, heparan sulfate, rhamnan sulfate, ketaran sulfate, alginic acid, hyaluronic acid, or carboxymethylcellulose.

12. The polymer substrate for removing a virus according to any one of Claims 1 to 11, wherein the virus is hepatitis B virus or hepatitis C virus.

13. A method for removing a virus, using the polymer substrate for removing a virus according to any one of Claims 1 to 12.

14. The method for removing a virus according to Claim 13, comprising mixing together a fluid having passed through pores of a porous hollow fiber and a fluid not having passed through the pores by passing a fluid containing a virus through the porous hollow fiber.

15. The method for removing a virus according to Claim 14, wherein the fluid containing a virus is blood containing a virus.

FIG. 1

## INTERNATIONAL SEARCH REPORT

| International application No. |
| --- |
| PCT/JP2012/055043 |

A.   CLASSIFICATION OF SUBJECT MATTER
*A61M1/36*(2006.01)i, *A61M1/34*(2006.01)i

According to International Patent Classification (IPC) or to both national classification and IPC

B.   FIELDS SEARCHED

Minimum documentation searched (classification system followed by classification symbols)
A61M1/36, A61M1/34, B01J20/00

Documentation searched other than minimum documentation to the extent that such documents are included in the fields searched
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
JSTPlus/JMEDPlus/JST7580(JDreamII)

C.   DOCUMENTS CONSIDERED TO BE RELEVANT

| Category* | Citation of document, with indication, where appropriate, of the relevant passages | Relevant to claim No. |
| --- | --- | --- |
| A | JP 2777604 B2   (Asahi Medical Co., Ltd.),<br>23 July 1998 (23.07.1998),<br>entire text; all drawings<br>(Family: none) | 1-12 |
| A | JP 7-289891 A  (Terumo Corp.),<br>07 November 1995 (07.11.1995),<br>entire text; all drawings<br>& US 5667684 A          & EP 679436 A1<br>& DE 69517466 T2 | 1-12 |
| A | JP 59-102436 A  (Kaneka Corp.),<br>13 June 1984 (13.06.1984),<br>entire text; all drawings<br>& US 4576928 A          & EP 464872 A1<br>& DE 3382723 T2 | 1-12 |

| ☒    Further documents are listed in the continuation of Box C. | ☐    See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 18 May, 2012 (18.05.12) | 29 May, 2012 (29.05.12) |

| Name and mailing address of the ISA/<br>Japanese Patent Office | Authorized officer |
| --- | --- |
| Facsimile No. | Telephone No. |

Form PCT/ISA/210 (second sheet) (July 2009)

## INTERNATIONAL SEARCH REPORT

| International application No. |
| --- |
| PCT/JP2012/055043 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2009-521413 A (Exthera AB.), 04 June 2009 (04.06.2009), entire text; all drawings & US 2009/0136586 A1 & WO 2007/069983 A1 & KR 10-2008-0077405 A & CN 101370536 A | 1-12 |
| A | JP 2003-135596 A (The Foundation for the Promotion of Industrial Science), 13 May 2003 (13.05.2003), entire text; all drawings (Family: none) | 1-12 |
| A | JP 2010-68910 A (DIC Corp. et al.), 02 April 2010 (02.04.2010), entire text; all drawings (Family: none) | 1-12 |

Form PCT/ISA/210 (continuation of second sheet) (July 2009)

<table>
<tr><td><b>INTERNATIONAL SEARCH REPORT</b></td><td>International application No.<br><br>PCT/JP2012/055043</td></tr>
</table>

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 13-15
   because they relate to subject matter not required to be searched by this Authority, namely:
   (See extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**     ☐ The additional search fees were accompanied by the applicant's protest and, where applicable, the payment of a protest fee.

    ☐ The additional search fees were accompanied by the applicant's protest but the applicable protest fee was not paid within the time limit specified in the invitation.

    ☐ No protest accompanied the payment of additional search fees.

Form PCT/ISA/210 (continuation of first sheet (2)) (July 2009)

**INTERNATIONAL SEARCH REPORT**

International application No.

PCT/JP2012/055043

Continuation of Box No.II-1 of continuation of first sheet(2)

Claims 13-15 pertain to a method for treatment of the human body by therapy since the inventions of claims 13-15 involves removal of virus from blood in a patient through extracorporeal circulation, and thus relate to a subject matter on which it is not required to carry out an international search under the provisions of PCT Article 17(2)(a)(i) and PCT Rule 39.1.

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005230165 A
- JP 10323387 A
- JP 2010068910 A
- JP 61271003 A
- JP 2029260 A

**Non-patent literature cited in the description**

- *Viral Hepatitis: Advances in Basic and Clinical Research,* 2011, vol. 69 (4
- **A. K. FUJIWARA et al.** *Hepatol. Res.,* 2007, vol. 37, 701
- **ZAHN, J. P. ALLAIN.** *J. Gen. Virol.,* 2005, vol. 86, 677